# EUROPÄISCHE PATENTSCHRIFT

(11) **EP 1 962 742 B1**
(45) Veröffentlichungstag und Bekanntmachung des Hinweises auf die Patenterteilung: **21.11.2012**
(21) Anmeldenummer: 06817495.2
(22) Anmeldetag: 21.12.2006
(51) Int. Cl.: A61F 5/00

(54) **SYSTEM ZUR KONTROLLE EINES STEUERBAREN MAGENBANDES**
SYSTEM FOR CONTROLLING A CONTROLLABLE STOMACH BAND
SYSTEME DE REGLAGE D'UN ANNEAU GASTRIQUE REGLABLE

(30) Priorität: 22.12.2005 AT 20582005
(43) Veröffentlichungstag der Anmeldung: 03.09.2008
(60) Teilanmeldung: 10164256.9
(73) Patentinhaber: Lechner, Wolfgang, 3441 Pixendorf (AT)
(72) Erfinder: Lechner, Wolfgang, 3441 Pixendorf (AT)
(74) Vertreter: Sonn & Partner Patentanwälte
(86) Internationale Anmeldenummer: PCT/AT2006/000529
(87) Internationale Veröffentlichungsnummer: WO 2007/070906

(56) Entgegenhaltungen:
- EP-A- 1 736 123
- WO-A-2006/113187
- WO-A-2006/118793
- WO-A-2006/122285
- WO-A1-01/12078

## Beschreibung

Die Erfindung betrifft ein System zur Kontrolle eines steuerbaren Magenbandes nach dem Oberbegriff des Anspruchs 1.

Steuerbare Magenbänder, bei welchen eine Zufuhr von Flüssigkeit in die Kammer bzw. Entleerung von Flüssigkeit aus der Kammer durch Anstechen einer subkutan fixierten Kammer, eines so genannten Ports, der mit der Kammer des Magenbandes über eine entsprechende Leitung verbunden ist, erreicht wird, werden von zahlreichen Firmen in prinzipiell gleicher Bauart angeboten. Bei einem derartigen Magenband handelt es sich um ein zur Restriktion der Nahrungsaufnahme und damit zur Gewichtsreduktion eingesetztes medizinisches Implantat, das um den obersten Magenteil herum geschlungen und verschlossen wird.

Die US 6 966 875 B1 beschreibt ein Magenband, welches gürtelförmig um den Magen herumgelegt und befestigt wird. Eine Einstellung der Einengung des Stomas ist rein mechanisch durch Einengung des Bandes möglich.

Die US 4 592 339 A beschreibt ein Magenband, bei dem an der dem Magen zugewandten Seite des Bandes eine Kammer angeordnet ist, die mit Flüssigkeit aufgefüllt werden kann. Dadurch ist die Steuerung der Stomaweite möglich. Über einen subkutan eingenähten Port, der über einen Schlauch mit der Kammer des Magenbandes verbunden ist, kann eine Flüssigkeitsfüllung und Entleerung des Systems durchgeführt werden.

Die WO 2004/014245 A1 beschreibt ein steuerbares Magenband, wobei die Verschiebung der Flüssigkeit von einem Port in die Kammer des Magenbandes ferngesteuert von außen vorgenommen werden kann.

Die EP 1 736 123 A1 stellt einen Stand der Technik nach Art. 54(3) EPÜ dar und beschreibt ein System zur Kontrolle eines steuerbaren Magenbands der gegenständlichen Art.

Neueste Forschungen haben gezeigt, dass dieser Bandinnendruck sehr exakt die Peristaltik der Speiseröhre wiedergibt. An Hand dieser Druckdaten lässt sich eine sehr exakte Einstellung der optimalen Stomaweite durchführen. Die Druckkurven geben auch Auskunft darüber, wie lange und wie oft gegessen bzw. getrunken wird. Daher ist der Bandinnendruck sehr gut für verschiedenste Kontrollzwecke in Zusammenhang mit der Anwendung von Magenbändern geeignet (W. Lechner et al: In Vivo Band Manometry: a New Access to Band Adjustment. Obesity Surgery, 15, 1432-1436, 2005).

Die derzeit verwendeten Magenbänder bringen in der Mehrzahl der Fälle sehr gute Langzeitergebnisse hinsichtlich Gewichtsreduktion und Patientenzufriedenheit. Dennoch gibt es einige Probleme die besonders bei hoher Bandauffüllung in den Vordergrund treten. Viele Patienten berichten über die unangenehme Erscheinung des Speichel-Erbrechens bzw. Herauswürgens, v.a. beim flachen Liegen. Speisereste können lange oberhalb des Stomas in der Speiseröhre verbleiben, hier zu gären beginnen und dadurch neben einem unangenehmen Mundgeruch eine Schleimhautreizung mit entsprechenden Schmerzen hervorrufen. Eine auf Dauer bestehende zu hohe Engstellung des Bandes führt im Verlauf von Monaten zu einer motorischen Erschöpfung der Speiseröhre die letztendlich in einer massiven Ausdehnung der Speiseröhre endet. Die geschluckten Speisen werden nicht mehr durch peristaltische Kontraktionswellen durch das Stoma in den Magen befördert sondern durch die Schwerkraft der sich oberhalb des Bandes auftürmenden geschluckten Speisen. Einen derartigen Zustand gilt es unbedingt zu verhindern, da damit auch der Verlust des Völlegefühls einhergeht. Die Bandwirkung geht dadurch verloren, was dann zu einer Gewichtszunahme trotz liegendem hoch aufgefülltem Magenband führt. Darüber hinaus ergeben sich verschiedenste mit der langen Verweildauer von Nahrungsbestandteilen im Ösophagus einhergehender Probleme wie retrosternales Brennen bzw. "Sodbrennens" und rezidivierende Aspiration von Nahrungsanteilen.

Die WO 2005/009305 A1 betrifft ein steuerbares Magenband, bei dem die oben genannten Probleme durch eine autoregulative Veränderung der Stomaweite bewältigt werden sollen. Sie gründen auf den Änderungen des Bandinnendruckes im Zusammenhang mit der Peristaltik der Speiseröhre während eines Essvorgangs. Der Bolus wird durch peristaltische Wellen durch das Magenband hindurch gedrückt. Dadurch wird ein Druckanstieg in der Kammer des Magenbandes verursacht, welcher zum Regeln des Druckes des Magenbandes herangezogen werden kann.

Die WO 01/12078 A1 zeigt ein Kontrollsystem der gegenständlichen Art, wobei die Druckwerte an der Magenwand erfasst werden und an eine Kontrolleinheit übermittelt werden. Bei Über- oder Unterschreitung von vordefinierten Werten des Druckes erfolgt eine entsprechende Änderung der Auffüllung des Magenbands. Die aktuellen Druckwerte an der Magenwand sind jedoch für eine unmittelbare Einstellung der Bandweite des Magenbands nicht geeignet.

Die US 6 475 136 B1 beschreibt ein steuerbares Magenband, bei welchem ein implantierter Drucksensor den Druck an der Magenwand ermittelt und zur Regelung der Bandeinstellung heranzieht.

Unter klinischen Bedingungen wurde der Druck im Magenband während eines Essvorgangs bereits erfasst. Für den Erhalt ausreichender Informationen müssen jedoch Messungen über zumindest eine halbe Stunde und dies in wiederkehrenden Zeitintervallen vorgenommen werden, was für den Patienten sehr zeitaufwändig und belastend ist. Darüber hinaus entstehen in der Regel nach der Aufnahme der Daten noch Wartezeiten bis die Daten vom behandelten Arzt ausgewertet und die zu setzenden Schritte definiert werden.

Alternativ oder zusätzlich dazu werden auch Röntgenuntersuchungen nach der Verabreichung eines Kontrastmittels vorgenommen, was wesentlich schneller als die in vivo Messung des Bandinnendruckes abläuft. Diese Messungen sind jedoch für den Patienten aufgrund der Röntgenstrahlung belastend und darüber hinaus sehr kostenaufwändig.

Die Aufgabe der vorliegenden Erfindung besteht daher in der Schaffung eines oben genannten Systems zur Kontrolle eines steuerbaren Magenbandes, mit welchem eine einfache und für den Patienten wenig belastende Überwachung des Druckes im Magenband möglich wird. Die vorliegende Erfindung soll die bisher auftretenden Probleme bei Magenbändern ohne autoregulative Funktion beheben bzw. reduzieren.

Gelöst wird die erfindungsgemäße Aufgabe dadurch, dass ein Speicher im Magenband angeordnet ist. Durch ein solches System wird es möglich, den Druck an der Magenwand während bestimmter Ereignisse, vorzugsweise während des Essens oder kontinuierlich zu erfassen und an eine Kontrolleinheit drahtlos zu übertragen. Dabei kann die Übertragung der erfassten Druckwerte permanent erfolgen oder es kann eine bestimmte Anzahl von Daten zwischengespeichert und auf Anforderung an eine Kontrolleinheit drahtlos übertragen werden. Der Patient muss sich zur Erfassung der Druckwerte nicht in ambulante Behandlung begeben, sondern es werden die wichtigen Daten während des normalen Tagesablaufs oder sogar der Nachtstunden gesammelt. Über die vorzugsweise kontinuierliche Überwachung des Druckes im Magenband können die Probleme derzeit verwendeter Magenbänder ohne autoregulative Funktion behoben werden. Der Druck, mit welchem das Magenband bzw. die Fluid-gefüllte Kammer an den Magen drückt, spielt eine Schlüsselrolle beim so genannten Gastric Banding. Die Bandinnendrucke spiegeln das Ausmaß der Abflussbehinderung und insbesondere die Rückwirkung der Abflussbehinderung auf die Motorik der Speiseröhre wieder. In dem zumindest einen Speicher können die Werte zwischengespeichert und bei Aufforderung zur Auswertung übertragen werden. Auch kann ein solcher Speicher zum Zwischenspeichern der Druckwerte im Fall einer Betriebsstörung dienen, so dass keine wichtige Information verloren geht. Die Ergebnisse liefern dem Patienten oder dem behandelnden Arzt oder dergl. wichtige Informationen. Durch das erfindungsgemäße System wird eine Kontrolle von physiologischen Parametern, welche in Zusammenhang mit "Gastric Banding" von Bedeutung sind, erzielt. Das Kontrollsystem ermöglicht eine einfache und für den Patienten wenig belastende Überwachung der motorischen Funktion der Speiseröhre. In der Folge von Schluckakten kommt es zu Druckanstiegen im Magenband. Die peristaltische Welle befördert den Bissen durch das Band in den Magen. Bei der Stomapassage kommt es zum erwähnten Druckanstieg im Bandsystem. Die Amplitude des Druckanstieges im Magenband hängt von der Amplitude der peristaltischen Welle, dem Ausmaß der Abflussbehinderung und der Viskosität des Bissens ab. Passiert der Bissen nicht vollständig das Band und bleibt daher ein Teil desselben in der Speiseröhre zurück, dann entstehen sekundäre peristaltische Wellen, d.h. peristaltische Wellen die nicht durch einen Schluckakt ausgelöst werden sondern durch die Dehnung der Speiseröhre durch den zurückgebliebenen Bissen. Es treten solange sekundäre peristaltische Wellen auf bis die Speiseröhre vom Bissen gereinigt ist. Diese Vorgänge können anhand des Bandinnendruckes mitverfolgt werden. Durch ein vorzugsweise kontinuierliches Erfassen des Druckverlaufes im Bandinneren kann die motorische Funktion der Speiseröhre exakt überwacht werden. Eine zu enge Bandeinstellung führt dazu, dass Speisereste sehr lange oberhalb des Bandes verbleiben, fortlaufend eine sekundäre peristaltische Aktivität auslösen, was letztendlich zur motorischen Erschöpfung der Speiseröhre führt. Genau das kann durch ein Druckmonitoring verhindert werden. Der Arzt kann bei der Bandengstellung bis an die Grenze des Verträglichen gehen und damit den maximalen Therapie-Effekt erzielen ohne eine Gefährdung der motorischen Aktivität der Speiseröhre in Kauf nehmen zu müssen. Durch das Druckmonitoring kann eine optimale Magenbandeinstellung aber auch eine Selbstkontrolle des Patienten hinsichtlich des Essverhaltens erreicht werden. Durch den Speicher im Magenband können die ermittelten Druckwerte zwischengespeichert und bei Aufforderung an die Kontrolleinheit zur Auswertung übertragen werden. Auch kann ein solcher Speicher im Magenband zum Zwischenspeichern der Druckwerte im Falle einer Betriebsstörung der Kontrolleinheit dienen, so dass keine wichtige Information verloren geht.

Die Übertragungseinrichtung ist vorzugsweise durch einen Hochfrequenzsender und die Empfangseinrichtung durch einen Hochfrequenzempfänger gebildet. Die Leistung des mitimplantierten Hochfrequenzsenders ist für die Erzielung relativ niedriger Reichweiten relativ gering, wodurch der Stromverbrauch ebenfalls minimal ist und somit die Lebensdauer des Implantats erhöht ist.

Ebenso ist es möglich, den Hochfrequenzsender durch einen passiven Sender zu bilden und die für die Übertragung der erfassten Druckwerte notwendige Energie von außen induktiv in das System einzukoppeln. Derartige passive Systeme sind bei anderen Implantaten bereits bekannt.

Um eine Bidirektionalität der Datenübertragung zwischen Kontrolleinheit und implantierten Magenband zu erzielen, kann die Übertragungseinrichtung im Magenband und die Empfangseinrichtung in der Kontrolleinheit durch einen Hochfrequenztransponder, welcher sowohl Sende- als auch Empfangsfunktion übernimmt, gebildet sein.

Gemäß einem weiteren Merkmal der Erfindung ist zumindest ein Drucksensor durch einen piezoelektrischen Sensor gebildet. Der Sensor kann an einer beliebigen Stelle im Magenband angeordnet sein, welche sich zur Messung des Druckes, welcher auf die Magenwand wirkt, eignet, also beispielsweise in der Kammer oder an der stomaseitig angeordneten Kammerwand oder dgl.

Ebenso kann ein Speicher zum Speichern der ermittelten Daten einerseits und allfälliger Berechnungsformeln oder Programmen andererseits in der Kontrolleinheit vorgesehen sein. Weiters ist in der Kontrolleinheit vorzugsweise eine Anzeige vorgesehen, über die dem Patienten die Daten bzw. daraus abgeleitete Daten übermittelt werden können. Dabei kann die Anzeige einerseits aus einfachen Leuchtmitteln bestehen, über die gewisse Zustände optisch dargestellt werden oder auch durch Ziffernanzeigen zur Darstellung der Druckwerte oder daraus abgeleiteter Werte bis hin zu Bildschirmen, über die der Verlauf des Druckes über der Zeit grafisch dargestellt werden kann.

Um eine zeitliche Zuordnung der Daten zu erzielen, kann in der Kontrolleinheit ein Zeitmodul vorgesehen sein, wie es bei Mikroprozessoranwendungen üblicherweise der Fall ist.

Zur Inbetriebnahme der Kontrolleinheit oder auch zur Umschaltung von Betriebszuständen kann in der Kontrolleinheit zumindest ein Bedienungselement vorgesehen sein.

Um dem Patienten oder dem behandelnden Arzt das Eintreten bestimmter Zustände oder dgl. anzuzeigen, kann in der Kontrolleinheit ein Signalgeber vorgesehen sein.

Ein solcher Signalgeber kann beispielsweise durch einen Lautsprecher oder einen Schwingungserzeuger gebildet sein. Letzterer hat den Vorteil, dass ähnlich wie beim Vibrieren eines Mobiltelefons die Umgebung nicht auf das Signal des Signalgebers aufmerksam gemacht wird.

Schließlich verfügt die Kontrolleinheit vorzugsweise über eine Schnittstelle, um die Daten beispielsweise an einen Computer oder dgl. zu übertragen. Auf diese Weise ist es auch möglich, dass der Patient die Daten zur Auswertung an die Klinik oder den behandelnden Arzt beispielsweise über Internet oder am Telefonnetz übermittelt und für eine Befundung nicht an die Klinik oder zum behandelnden Arzt muss.

Vorteilhafterweise ist das Gehäuse der Kontrolleinheit in Form einer Armbanduhr mit einem entsprechenden Uhrband gebildet. Auf diese Weise kann der Patient mit dem implantierten Magenband ständig die Kontrolleinheit in bequemer Art und Weise bei sich tragen und wird somit ständig über den Zustand des Magenbandes informiert. Ebenso können die Daten über einen bestimmten Zeitraum, beispielsweise 24 Stunden, gespeichert werden und danach die Kontrolleinheit an eine Auswerteeinrichtung, beispielsweise beim behandelnden Arzt, übermittelt werden, wo eine Analyse der Daten stattfindet.

Weiters kann eine Einrichtung zur Einbringung oder Absaugung des Fluids in oder aus der Kammer des Magenbandes vorgesehen sein, so dass aufgrund der erfassten und ausgewerteten Druckwerte der Druck in der Kammer des Magenbandes optimierbar ist. Auf diese Weise kann ein automatisches oder halbautomatisches System geschaffen werden, welches aufgrund der erfassten Druckwerte das Magenband immer optimal einstellt. Die Einrichtung zur Einbringung oder Absaugung des Fluids in oder aus der Kammer kann dabei ebenfalls durch ein Implantat gebildet sein, welches die entsprechende Information drahtlos von der Kontrolleinheit übermittelt bekommt.

Die Einrichtung zur Einbringung oder Absaugung des Fluids kann durch eine in der Verbindung zwischen der Kammer und der zweiten Kammer des Magenbandes angeordneten Pumpe gebildet sein, welche das Fluid aus der Kammer in die zweite Kammer oder umgekehrt befördern kann.

Zusätzlich kann eine wichtige Information über das Essverhalten des Patienten mit Hilfe eines Sensors zur Messung der Schluckaktivität geschaffen werden, welcher Sensor mit einer Einrichtung zur drahtlosen Übertragung der erfassten Sensorwerte an die Kontrolleinheit verbunden ist. Ein derartiger Schlucksensor kann beispielsweise durch ein Mikrofon oder einen Druckwandler, der an den Hals des Patienten aufgeklebt wird, gebildet sein.

Gemäß einem weiteren Merkmal der Erfindung ist eine Leitung zur Verbindung der Kontrolleinheit mit einer Kommunikationseinrichtung, insbesondere einem Computer oder einem Telefon, vorgesehen. Auf diese Weise kann über die Schnittstelle der Kontrolleinheit und die besagte Leitung eine Datenverbindung zu einer Kommunikationseinrichtung und von dort zu weiteren Kommunikationseinrichtungen, beispielsweise im Internet oder einem Telefonnetz, hergestellt werden.

Die zweite Kammer des Magenbandes ist üblicherweise bei den gegenständlichen Magenbändern ohne autoregulative Funktion, üblicherweise durch einen subkutan anzuordnenden Port gebildet, über welchen mit Hilfe einer Subkutanspritze das Fluid in die Kammer des Magenbandes eingebracht oder aus dieser abgesaugt werden kann.

Die beiden Kammern des steuerbaren Magenbandes bzw. die Kammer und der Port, sind vorzugsweise über eine Leitung miteinander verbunden, wobei in der Leitung zumindest eine Einrichtung zur Steuerung des Durchflusses des Fluids angeordnet sein kann. Diese Durchflusssteuerungseinrichtung kann beispielsweise durch ein Ventil oder eine Pumpe gebildet sein, wodurch eine automatische Regelung des Druckes in der Kammer des Magenbandes und somit auf die Magenwand aufgrund der gemessenen Druckwerte ermöglicht wird.

Vorteilhafterweise ist die Verarbeitungseinrichtung zur Ermittlung der Dauer und Geschwindigkeit eines Essvorganges ausgebildet. Aufgrund der ermittelten Parameter über den Essvorgang, kann eine Optimierung der Bandeinstellung durch den Arzt oder automatisch erfolgen oder dem Patienten wichtige Information über sein Essverhalten geliefert werden.

Dabei ist die Verarbeitungseinrichtung vorzugsweise zur Auswertung der Amplitude und Dauer der ermittelten Druckwerte während eines Essvorganges ausgebildet.

Die Amplitude und Dauer der Anstiege der Druckwerte sind wichtige Indikatoren für eine zu starke Belastung des Ösophagus.

Die Erfindung wird anhand der folgenden Abbildungen näher erläutert. Darin zeigen:
- Fig. 1: eine schematische Darstellung eines Kontrollsystems bestehend aus dem Magenband mit dem Sensor und der Kontrolleinheit;
- Fig. 2: eine schematische Darstellung eines implantierten erfindungsgemäßen Magenbandes mit einer Kontrolleinheit in Form einer Armbanduhr;
- Fig. 3: eine schematische Darstellung der Datenübertragung von einer Kontrolleinheit beispielsweise zu einem Computer;
- Fig. 4: den zeitlichen Verlauf des erfassten Druckes im Magenband während mehrerer Schluckvorgänge bei verschiedenen Füllungen der Kammer des Magenbandes; und
- Fig. 5: den zeitlichen Verlauf des Druckes im Magenband während eines Essvorganges.

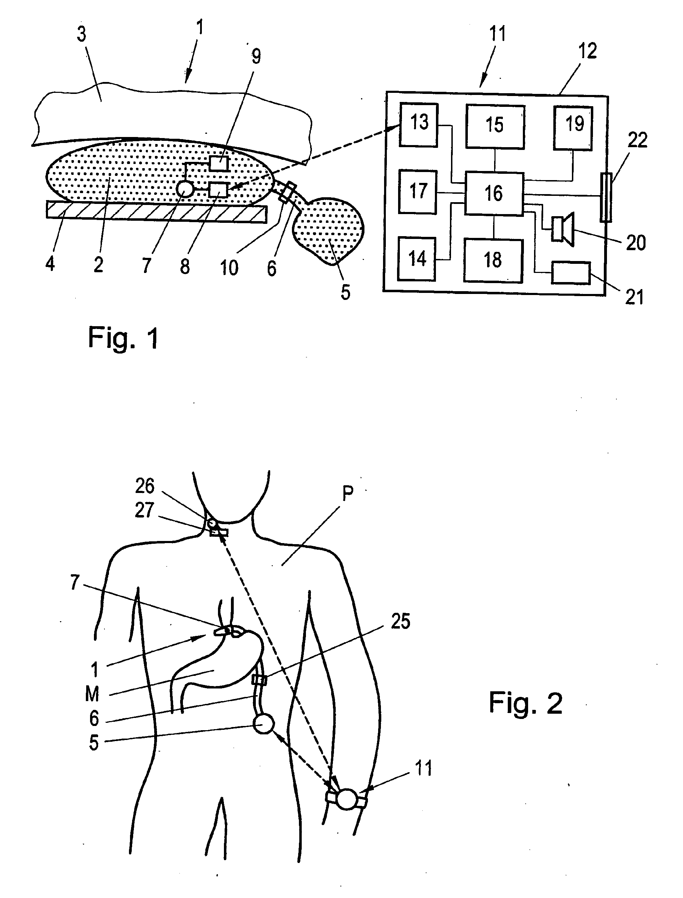

Fig. 1 zeigt schematisch ein Magenband 1 zusammen mit einer Kontrolleinheit 11, mit Hilfe welcher der Druck des Magenbandes 1 überwacht werden kann. Das Magenband 1 besitzt einen nicht dehnbaren Rücken 4. An der der Magenwand 3 zugewandten Seite des Rückens 4 befindet sich zumindest eine Kammer 2, welche mit einer Flüssigkeit bzw. einem Fluid befüllbar ist. Die Kammer 2 ist mit einer zweiten Kammer 5 über eine Leitung 6 verbunden. Durch Verschiebung der Flüssigkeit zwischen den Kammern 2 und 5 kann die Einengung des um den Mageneingang gelegten Magenbandes 1 und somit der Druck auf den Magen verändert werden. Die zweite Kammer 5 kann als subkutan im Unterbauch angeordneter so genannter Port ausgebildet sein (siehe Fig. 2), über welchen von außen mittels einer Subkutanspritze Flüssigkeit zu- oder abgeführt und somit die Flüssigkeitsmenge und in der Folge der Druck in der Kammer 2 verändert werden kann. Zur Steuerung des Durchflusses der Flüssigkeit bzw. des Fluids von der einen Kammer 2 zur anderen Kammer 5 bzw. umgekehrt kann auch in der Verbindungsleitung 6 eine entsprechende Einrichtung 10 zur Steuerung des Durchflusses der Flüssigkeit vorgesehen sein. Diese Steuerungseinrichtung 10 kann beispielsweise durch ein Ventil oder eine Pumpe gebildet sein (nicht dargestellt). In der Kammer 2 bzw. an einer anderen geeigneten Stelle, wie zum Beispiel in der zweiten Kammer 5, ist zumindest ein Sensor 7 zur Messung des auf die Magenwand 3 wirkenden Druckes bzw. einer dazu proportionalen Größe vorgesehen, welcher Sensor 7 mit einer Einrichtung 8 zur drahtlosen Übertragung der erfassten Druckwerte an eine Kontrolleinheit 11 verbunden ist. Zum Speichern bzw. Zwischenspeichern der vom Drucksensor 7 erfassten Druckwerte kann ein Speicher 9 vorgesehen sein. Die implantierte Übertragungseinrichtung 8 kann durch eine Batterie mit elektrischer Energie versorgt werden, oder vollständig passiv aufgebaut sein, wobei die für die Übertragung der Daten notwendige Energie von außen, insbesondere vom Empfänger der Kontrolleinheit 11, eingebracht werden würde. Um eine Datenübertragung nicht nur vom Magenband 1 zur Kontrolleinheit 11 sondern auch umgekehrt zuzulassen, kann die Übertragungseinrichtung 8 auch durch einen Transponder gebildet sein.

Die Kontrolleinheit 11 beinhaltet eine Einrichtung 13 zum Empfang der übertragenen Druckdaten des Sensors 7 des Magenbandes 1, die ebenfalls als Transponder ausgebildet sein kann. Neben einem Speicher 14 zum Speichern der übertragenen Daten aber auch von Programmen ist vorzugsweise eine Anzeige 15 im Gehäuse 12 der Kontrolleinheit 11 angeordnet. Über eine Einrichtung 16 werden die empfangenen Druckwerte verarbeitet. Diese Verarbeitungseinrichtung 16 ist durch einen Mikroprozessor gebildet. Um die erfassten und übermittelten Daten zeitlich zuordnen zu können, kann ein Zeitmodul 17 mit der Verarbeitungseinrichtung 16 verbunden sein. Weiters kann für die Inbetriebnahme der Kontrolleinheit 11 aber auch zum Umschalten von Betriebszuständen zumindest ein Bedienungselement 18 vorgesehen sein.

Um den Patienten aber auch den Arzt über das Eintreten bestimmter Zustände zu informieren, kann ein Signalgeber, beispielsweise ein Lautsprecher 20 oder ein Schwingungserzeuger 21 vorgesehen sein. Die Komponenten der Kontrolleinheit 11 werden von einer Spannungsversorgung 19, welche vorzugsweise durch eine wiederaufladbare Batterie gebildet ist, mit elektrischer Energie versorgt.

Um die im Speicher 14 gespeicherten Daten beispielsweise an einen Computer übertragen zu können, kann eine Schnittstelle 22 vorgesehen sein. Über eine derartige Schnittstelle 22 können auch Daten in die Verarbeitungseinrichtung 16 oder in den Speicher 14 geschrieben werden. Die Schnittstelle 22 kann beispielsweise durch verschiedene standardisierte Schnittstellen gebildet sein. Dabei kommen sowohl leitungsgebundene als auch drahtlose (z.B. Infrarot- oder Hochfrequenz-) Schnittstellen zur Anwendung.

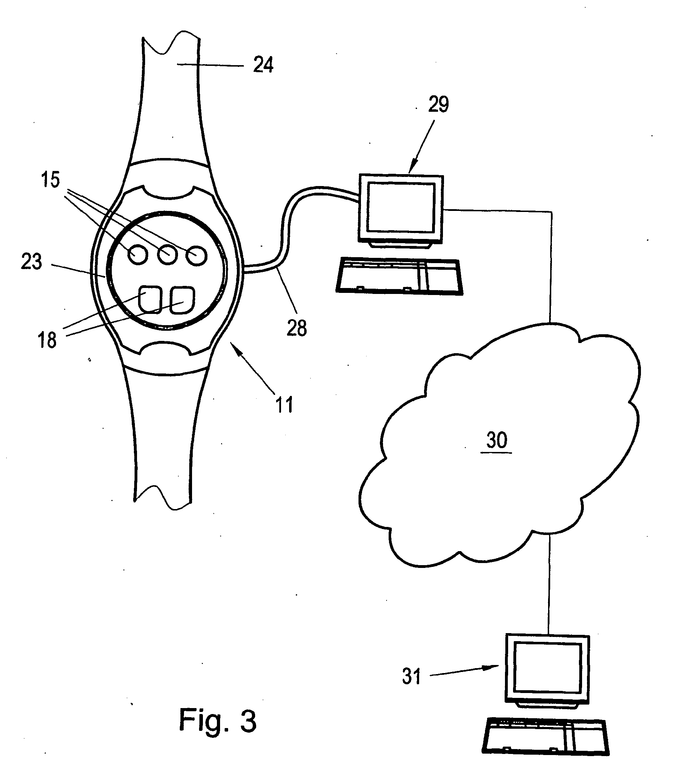

Fig. 2 zeigt schematisch eine Anwendung des erfindungsgemäßen Magenbandes 1 mit zumindest einem Drucksensor 7, bei welchem die zweite Kammer 5 durch einen so genannten subkutanen Port gebildet ist, der mit der Kammer 2 des Magenbandes 1 über eine Leitung 6 verbunden ist, in welcher beispielsweise eine Pumpe 25 angeordnet sein kann, welche den Flüssigkeitstransport von der Kammer 2 zur Kammer 5 oder umgekehrt steuert. Das Magenband 1 umschließt den Eingang des Magens M des Patienten P. Erfindungsgemäß werden die Daten des Drucksensors 7 an die Kontrolleinheit 11, welche im dargestellten Beispiel in Form einer Armbanduhr mit einem entsprechenden Uhrband (s. Fig. 3) gebildet ist, übermittelt, wo sie verarbeitet und gespeichert bzw. angezeigt werden. Zusätzlich zum Drucksensor 7 im Magenband 1 kann ein Sensor 26 zur Messung der Schluckaktivität des Patienten P mit einer entsprechenden Einrichtung 27 zur drahtlosen Übertragung verbunden sein und können diese Daten ebenfalls drahtlos an die Kontrolleinheit 11 übermittelt werden. Für den Patienten P dient die Kontrolleinheit 11 als Biofeedback-Instrument, welches sein Essverhalten wiederspiegelt. Neben der Dauer der Nahrungsaufnahme können auch andere Faktoren, wie die Geschwindigkeit der Nahrungsaufnahme und das Ausmaß des Kauens ermittelt und angezeigt werden. Eine große Essgeschwindigkeit führt genauso wie schlechtes Kauen zu höheren Druckanstiegen im Magenband. Damit wird das Druckmonitoring für den Patienten zu einem Instrument der Selbstkontrolle hinsichtlich des Essverhaltens. Darüber hinaus können Störungen des Systems, z.B. durch Flüssigkeitsverlust wegen Undichtwerden des Magenbandes 1 sofort erkannt und dem Patienten P rückgemeldet werden.

Für den Träger des Magenbandes 1 ist eine Änderung der Esstechnik erforderlich, um unangenehme Sensationen bzw. ein Erbrechen zu verhindern. Es ist ein langsames Essen mit gutem Kauen sowie die Einhaltung von entsprechenden Pausen zwischen den einzelnen Bissen erforderlich. Der hochgradig adipöse Patient mit fast immer entsprechend massiver Essstörung tut sich oft schwer auf diese richtige Esstechnik umzustellen. Eine fortwährend beibehaltene falsche Esstechnik kann den Langzeit-Therapieerfolg mit dem Magenband beeinträchtigen und zu einer schweren motorischen Störung der Ösophagusperistaltik bzw. einer massiven Ausdehnung der Speiseröhre führen. Daher erscheint es als sehr vorteilhaft, dem Patienten mit Hilfe der Kontrolleinheit 11 eine ständige Rückmeldung zu geben, ob er die Nahrungsaufnahme in der richtigen Art und Weise durchführt und ob er zu lange isst.

Die Kontrolleinheit 11 bereitet die Druckdaten in einer für den Patienten unmittelbar einsichtigen Form auf und gibt ihm sofortige Rückmeldungen hinsichtlich seines Essverhaltens sowie Warnhinweise bei nicht einwandfreiem Funktionieren des Magenbandes 1 sowie bei Zeichen der muskulären Erschöpfung der Speiseröhre. Er wird dann auf die Notwendigkeit einer sofortigen ärztlichen Kontrolle hingewiesen. Die Rückmeldungen an den Patienten P können über optische Informationen auf einer Anzeige 15 oder durch akustische oder taktile Signalgeber übermittelt werden (s. Fig. 1).

Fig. 3 zeigt schematisch ein Blockschaltbild über die Datenfernübertragung von der Kontrolleinheit 11 beispielsweise zu einem Computer 31. In diesem Fall wird die Kontrolleinheit 11, vorzugsweise über die Schnittstelle 22 (s. Fig. 1), mit einer entsprechenden Leitung 28 mit einer Kommunikationseinrichtung, beispielsweise einem Computer 29, verbunden. Auf diese Weise können die in der Kontrolleinheit 11 enthaltenen Daten an den Computer 29 und von diesem über ein Datennetz 30, insbesondere das Internet, zu einem weiteren Computer 31 übermittelt werden. Auf diese Weise kann der Patient die Daten seiner Kontrolleinheit 11 in bequemer und zeitsparender Weise an einen Computer 31 der Klinik oder des behandelnden Arztes überweisen, wo diese entsprechend ausgewertet werden.

Die Optimierung der Bandeinstellung ist ein wichtiger Punkt, um einerseits einen maximalen Therapieerfolg zu erreichen, und um andererseits Schädigungen der Speiseröhren durch ein zu langes Verweilen von Speisen im Ösophagus zu verhindern (siehe oben). Das Zurückbleiben von Anteilen des Speisebreis in der Speiseröhre regt das Auftreten von sekundären peristaltischen Wellen an. Diese sekundäre Peristaltik versucht den Bolus weiter zu befördern und die Speiseröhre zu reinigen. Gelingt dies nicht und bleiben Speisereste ständig oberhalb des Magenbandes liegen, führt dies im Verlauf von Tagen und Wochen einerseits zu einer Schleimhautentzündung mit entsprechenden Schmerzen, andererseits zu einer motorischen Erschöpfung und zunehmenden Ausdehnung der Speiseröhre.

Für den Arzt ist daher die Kontrolle der von ihm durchgeführten Bandeinstellung von großer Bedeutung. Die Qualität der Einstellung erweist sich jedoch erst im Tagesablauf im Rahmen der Nahrungsaufnahmen und der Schlafphasen. Dann zeigt sich erst, ob die gewählte Bandeinstellung zu einem zu langen postprandialen Verweilen von Nahrungsresten in der Speiseröhre führt bzw. während der Nachtruhe zu einem schlechten Abfließen des Speichels, etc. Daher empfiehlt sich zum Zwecke der optimalen Bandadjustierung die Aufzeichnung der Druckdaten über einen eingeschränkten Zeitraum, etwa über 24 Stunden. An Hand dieser Daten, die nunmehr sehr bequem erfasst und übertragen werden können, kann dann eine sehr exakte Nachadjustierung des Bandes durch den Arzt vorgenommen werden.

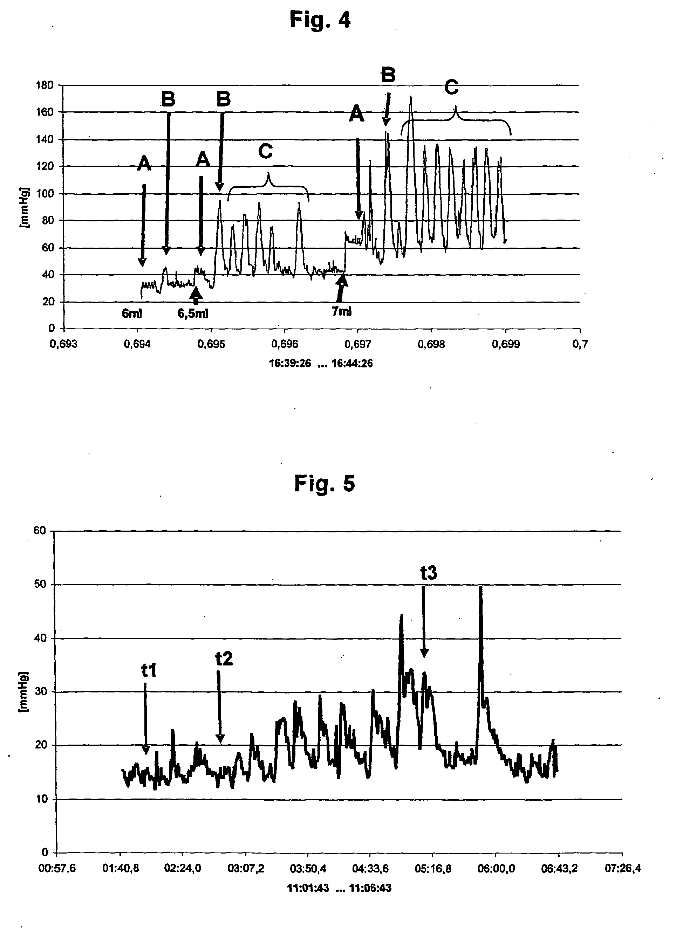

Fig. 4 zeigt den zeitlichen Verlauf des Druckes im Magenband während mehrerer Schluckvorgänge von jeweils 15 ml Wasser bei drei verschiedenen Bandfüllungen, nämlich 6 ml, 6,5 ml und 7 ml. Dieses Zeit-Druckdiagramm zeigt die Abhängigkeit der sekundären Peristaltik vom Ausmaß der Engstellung des Bandes und damit der Abflussbehinderung. Bei jeder Bandfüllung hat der Patient einen 15 ml Wasserschluck durchzuführen (Pfeil A). Bei 6 ml Bandfüllung zeigt sich nur ein einzelner Druckanstieg im Magenband. Dieser Druckanstieg entspricht der durch den Schluckakt ausgelösten primären peristaltischen Welle (Pfeil B). Bei 6,5 ml Adjustierung des Bandes folgen auf die primäre peristaltische Welle fünf sekundäre peristaltische Wellen (Bereich C) (nicht durch einen Schluckakt ausgelöst). Dies weist auf die bereits sehr ausgeprägte Abflussbehinderung hin. Mit 7 ml Füllvolumen ist das Band nicht mehr durchgängig. Dies drückt sich in der fortgesetzten sekundären Peristaltik (Bereich C) im Anschluss an den 15 ml Wasser-Schluckakt aus. Die optimale Bandfüllung liegt in diesem Fall im Bereich zwischen 6 und 6,5 ml.

Fig. 5 zeigt schließlich den zeitlichen Verlauf des erfassten Druckes in einem Magenband während eines aus Suppe und Dillkartoffeln zusammengesetzten Essens. Zum Zeitpunkt t1 beginnt der Proband mit dem Essen der Suppe, zum Zeitpunkt t2 mit dem Essen der Kartoffel. Der Pfeil mit der Bezeichnung t3 markiert die Beendigung des Essens. Die im Bandinneren abgeleiteten Druckwellen geben die Dauer des Essens wieder. Nach Beendigung des Essens können in Abhängigkeit vom Ausmaß der Abflussbehinderung im Bandinneren noch für unterschiedliche Zeit Druckwellen abgeleitet werden.

Abschließend sei erwähnt, dass aus den zeitlichen Druckverläufen durch die anatomische Nähe des Sensors 7 zum Herzen auch die Herzkontraktionen erkennen und damit den Herzrhythmus ableiten kann.

## Patentansprüche

1. System zur Kontrolle eines steuerbaren Magenbandes (1), mit einer Kontrolleinheit (11), wobei das Magenband (1) einen nicht dehnbaren Rücken (4) und eine stomaseitig vom Rücken (4) angeordnete, mit einem Fluid befüllbare Kammer (2), welche mit einer zweiten Kammer (5) in Verbindung steht, und zumindest einen Sensor (7) zur Erfassung des Druckes an der Magenwand (3) aufweist, und der Sensor (7) mit einer Einrichtung (8) zur drahtlosen Übertragung der erfassten Druckwerte an die Kontrolleinheit (11) verbunden ist, und die Kontrolleinheit (11) eine Einrichtung (13) zum Empfang der übertragenen Druckdaten des zumindest einen Sensors (7) des Magenbandes (1) sowie ein Gehäuse (12) aufweist und zumindest ein Speicher (9, 14) zum Speichern der ermittelten Druckwerte und eine durch einen Mikroprozessor gebildete Einrichtung (16) zur Verarbeitung der zeitlichen Verläufe der empfangenen Druckwerte vorgesehen ist, **dadurch gekennzeichnet, dass** ein Speicher (9) im Magenband (1) angeordnet ist.

2. Kontrollsystem nach Anspruch 1, **dadurch gekennzeichnet, dass** die Übertragungseinrichtung (8) durch einen Hochfrequenzsender und die Empfangseinrichtung (13) durch einen Hochfrequenzempfänger gebildet ist.

3. Kontrollsystem nach Anspruch 2, **dadurch gekennzeichnet, dass** der Hochfrequenzsender durch einen passiven Sender gebildet ist.

4. Kontrollsystem nach einem der Ansprüche 1 bis 3, **dadurch gekennzeichnet, dass** die Übertragungseinrichtung (8) und die Empfangseinrichtung (13) durch einen Hochfrequenztransponder gebildet ist.

5. Kontrollsystem nach einem der Ansprüche 1 bis 4, **dadurch gekennzeichnet, dass** zumindest ein Drucksensor (7) durch einen piezoelektrischen Sensor gebildet ist.

6. Kontrollsystem nach einem der Ansprüche 1 bis 5, **dadurch gekennzeichnet, dass** ein Speicher (14) in der Kontrolleinheit (11) angeordnet ist.

7. Kontrollsystem nach einem der Ansprüche 1 bis 6, **dadurch gekennzeichnet, dass** in der Kontrolleinheit (11) eine Anzeige (15) vorgesehen ist.

8. Kontrollsystem nach einem der Ansprüche 1 bis 7, **dadurch gekennzeichnet, dass** in der Kontrolleinheit (11) ein Zeitmodul (17) vorgesehen ist.

9. Kontrollsystem nach einem der Ansprüche 1 bis 8, **dadurch gekennzeichnet, dass** in der Kontrolleinheit (11) zumindest ein Bedienungselement (18) vorgesehen ist.

10. Kontrollsystem nach einem der Ansprüche 1 bis 9, **dadurch gekennzeichnet, dass** in der Kontrolleinheit (11) ein Signalgeber vorgesehen ist.

11. Kontrollsystem nach Anspruch 10, **dadurch gekennzeichnet, dass** der Signalgeber durch einen Lautsprecher (20) gebildet ist.

12. Kontrollsystem nach Anspruch 10, **dadurch gekennzeichnet, dass** der Signalgeber durch einen Schwingungserzeuger (21) gebildet ist.

13. Kontrollsystem nach einem der Ansprüche 1 bis 12, **dadurch gekennzeichnet, dass** in der Kontrolleinheit (11) eine Schnittstelle (22) vorgesehen ist.

14. Kontrollsystem nach einem der Ansprüche 1 bis 13, **dadurch gekennzeichnet, dass** das Gehäuse (12) der Kontrolleinheit (11) in Form einer Armbanduhr (23) mit einem entsprechenden Uhrband (24) gebildet ist.

15. Kontrollsystem nach einem der Ansprüche 1 bis 14, **dadurch gekennzeichnet, dass** eine Einrichtung zur Einbringung oder Absaugung des Fluids in oder aus der Kammer (2) des Magenbandes (1) vorgesehen ist, so dass aufgrund der erfassten und ausgewerteten Druckwerte der Druck in der Kammer (2) optimierbar ist.

16. Kontrollsystem nach Anspruch 15, **dadurch gekennzeichnet, dass** die Einrichtung zur Einbringung oder Absaugung des Fluids durch eine in der Verbindung (6) zwischen der Kammer (2) und der zweiten Kammer (5) angeordneten Pumpe (25) gebildet ist.

17. Kontrollsystem nach einem der Ansprüche 1 bis 16, **dadurch gekennzeichnet, dass** ein Sensor (26) zur Messung der Schluckaktivität vorgesehen ist, welcher Sensor (26) mit einer Einrichtung (27) zur drahtlosen Übertragung der erfassten Sensorwerte an die Kontrolleinheit (11) verbunden ist.

18. Kontrollsystem nach einem der Ansprüche 1 bis 17, **dadurch gekennzeichnet, dass** eine Leitung (28) zur Verbindung der Kontrolleinheit (11) mit einer Kommunikationseinrichtung, insbesondere einem Computer (29) oder einem Telefon vorgesehen ist.

19. Kontrollsystem nach einem der Ansprüche 1 bis 18, **dadurch gekennzeichnet , dass** die zweite Kammer (5) des Magenbandes (1) durch einen subkutan anzuordnenden Port gebildet ist.

20. Kontrollsystem nach einem der Ansprüche 1 bis 19, **dadurch gekennzeichnet, dass** die Kammern (2, 5) des Magenbandes über eine Leitung (6) miteinander verbunden sind, wobei in der Leitung (6) zumindest eine Einrichtung (10) zur Steuerung des Durchflusses des Fluids vorgesehen ist.

21. Kontrollsystem nach einem der Ansprüche 1 bis 20, **dadurch gekennzeichnet, dass** die Verarbeitungseinrichtung (16) zur Ermittlung der Dauer und Geschwindigkeit eines Essvorganges ausgebildet ist.

22. Kontrollsystem nach einem der Anspruch 1 bis 21, **dadurch gekennzeichnet, dass** die Verarbeitungseinrichtung (16) zur Auswertung der Amplitude und Dauer der ermittelten Druckwerte während eines Essvorganges ausgebildet ist.

## Claims

1. System for controlling a controllable gastric band (1), comprising a control unit (11), wherein the gastric band (1) includes a nonextensible back (4) and a chamber (2) arranged on the stoma side of the back (4) and capable of being filled with a fluid, which chamber is in connection with a second chamber (5) and has at least one sensor (7) for the detection of the pressure on the gastric wall (3), and wherein the sensor (7) is connected with a device (8) for the wireless transmission of the detected pressure values to the control unit (11), and wherein the control unit (11) includes a device (13) for receiving transmitted pressure data from the at least one sensor (7) of the gastric band (1), as well as a housing (12) and at least one memory (9, 14) for storing the pressure values detected and a device (16) formed by a microprocessor for processing the time courses of the pressure values received, **characterized in that** a memory (9) is arranged in the gastric band.

2. Control system according to claim 1, **characterized in that** the transmission device (8) is comprised of a high-frequency transmitter and that the receiving device (13) is comprised of a high-frequency receiver.

3. Control system according to claim 2, **characterized in that** the high-frequency transmitter is comprised of a passive transmitter.

4. Control system according to any one of claims 1 to 3, **characterized in that** the transmission device (8) and the receiving device (13) are comprised of a high-frequency transponder.

5. Control system according to any one of claims 1 to 4, **characterized in that** at least one pressure sensor (7) is comprised of a piezoelectric sensor.

6. Control system according to any one of claims 1 to 5, **characterized in that** a memory (14) is arranged in the control unit (1).

7. Control system according to any one of claims 1 to 6, **characterized in that** a display (15) is provided in the control unit (11).

8. Control system according to any one of claims 1 to 7, **characterized in that** a time module (17) is provided in the control unit (11).

9. Control system according to any one of claims 1 to 8, **characterized in that** at least one operating element (18) is provided in the control unit (11).

10. Control system according to any one of claims 1 to 9, **characterized in that** a signal generator is provided in the control unit (11).

11. Control system according to claim 10, **characterized in that** the signal generator is comprised of a loudspeaker (20).

12. Control system according to claim 10, **characterized in that** the signal generator is comprised of an oscillation generator (21).

13. Control system according to any one of claims 1 to 12, **characterized in that** an interface (22) is provided in the control unit (11).

14. Control system according to any one of claims 1 to 13, **characterized in that** the housing (12) of the control unit (11) is in the form of a watch (23) with an appropriate watch strap (24).

15. Control system according to any one of claims 1 to 14, **characterized in that** a device for introducing or sucking off fluid into or from the chamber (2) of the gastric band (1) is provided to enable the optimization of the pressure in the chamber (2) on the basis of the detected and evaluated pressure values.

16. Control system according to claim 15, **characterized in that** the device for introducing or sucking off fluid is comprised of a pump (25) arranged in the connection (6) between the chamber (2) and the second chamber (5).

17. Control system according to any one of claims 1 to 16, **characterized in that** a sensor (26) for measuring the swallowing activity is provided, which sensor (26) is connected with a device (27) for the wireless transmission of the detected sensor values to the control unit (11).

18. Control system according to any one of claims 1 to 17, **characterized in that** a line (28) for connecting the control unit (11) with a communication device, in particular a computer (29) or a telephone, is provided.

19. Control system according to any one of claims 1 to 18, **characterized in that** the second chamber (5) of the gastric band (1) is comprised of a port to be arranged subcutaneously.

20. Control system according to any one of claims 1 to 19, **characterized in that** the chambers (2, 5) of the gastric band are connected with each other via a duct (6), wherein at least one device (10) for controlling the flow rate of the fluid is provided in the duct (6).

21. Control system according to any one of claims 1 to 20, **characterized in that** the processing device (16) is configurated for detecting the duration and speed of an eating process.

22. Control system according to any one of claims 1 to 21, **characterized in that** the processing device (16) is configured for evaluating the amplitude and the duration of the detected pressure values during an eating process.

## Revendications

1. Système destiné au suivi d'un anneau gastrique (1) ajustable, comprenant une unité de suivi (11), système dans lequel l'anneau gastrique (1) comprend un dosseret (4) non extensible et une chambre (2) pouvant être remplie par un fluide, agencée du côté du dosseret (4) dirigé vers l'estomac, et qui est en communication avec une deuxième chambre (5), l'anneau gastrique comprenant en outre au moins un capteur (7) destiné à relever la pression au niveau de la paroi stomacale (3) et le capteur (7) étant en liaison avec un dispositif (8) pour la transmission sans fil, à l'unité de suivi (11), des valeurs de pression ayant été relevées, et système dans lequel l'unité de suivi (11) comprend un dispositif (13) destiné à la réception des données de pression ayant été transmises dudit au moins un capteur (7) de l'anneau gastrique (1), ainsi qu'un boitier 12), au moins une mémoire (9, 14) destinée à mémoriser les valeurs de pression déterminées et un dispositif de données (16) pour le traitement des lois de variation dans le temps des valeurs de pression reçues étant en outre prévus dans le système,
**caractérisé en ce qu'**une mémoire (9) est agencée dans l'anneau gastrique (1).

2. Système de suivi selon la revendication 1, **caractérisé en ce que** le dispositif de transmission (8) est formé par un émetteur haute-fréquence, et le dispositif de réception (13) par un récepteur haute-fréquence.

3. Système de suivi selon la revendication 2, **caractérisé en ce que** l'émetteur haute-fréquence est formé par un émetteur passif.

4. Système de suivi selon l'une des revendications 1 à 3, **caractérisé en ce que** le dispositif de transmission (8) et le dispositif de réception (13) sont formés par un transpondeur haute-fréquence.

5. Système de suivi selon l'une des revendications 1 à 4, **caractérisé en ce qu'**au moins un capteur de pression (7) est formé par un capteur piézoélectrique.

6. Système de suivi selon l'une des revendications 1 à 5, **caractérisé en ce qu'**une mémoire (14) est agencée dans l'unité de suivi (11).

7. Système de suivi selon l'une des revendications 1 à 6, **caractérisé en ce que** dans l'unité de suivi (11) est prévu un élément d'affichage (15).

8. Système de suivi selon l'une des revendications 1 à 7, **caractérisé en ce que** dans l'unité de suivi (11) est prévu un module d'horloge (17).

9. Système de suivi selon l'une des revendications 1 à 8, **caractérisé en ce que** dans l'unité de suivi (11) est prévu au moins un élément de commande de service (18).

10. Système de suivi selon l'une des revendications 1 à 9, **caractérisé en ce que** dans l'unité de suivi (11) est prévu un générateur de signal.

11. Système de suivi selon la revendication 10, **caractérisé en ce que** le générateur de signal est formé par un haut-parleur (20).

12. Système de suivi selon la revendication 10, **caractérisé en ce que** le générateur de signal est formé par un vibreur (21).

13. Système de suivi selon l'une des revendications 1 à 12, **caractérisé en ce que** dans l'unité de suivi (11) est prévue une interface (22).

14. Système de suivi selon l'une des revendications 1 à 13, **caractérisé en ce que** le boitier (12) de l'unité de suivi (11) est réalisé sous la forme d'une montre-bracelet (23) avec un bracelet de montre (24) correspondant.

15. Système de suivi selon l'une des revendications 1 à 14, **caractérisé en ce qu'**il est prévu un dispositif pour introduire ou aspirer le fluide dans ou de la chambre (2) de l'anneau gastrique (1), de sorte qu'il est possible d'optimiser la pression dans la chambre (2) sur la base des valeurs de pression relevées et traitées.

16. Système de suivi selon la revendication 15, **caractérisé en ce que** le dispositif pour l'introduction ou l'aspiration du fluide est formé par une pompe (25) agencée dans la liaison de communication (6) entre la chambre (2) et la deuxième chambre (5).

17. Système de suivi selon l'une des revendications 1 à 16, **caractérisé en ce qu'**il est prévu un capteur (26) pour mesurer l'activité de déglutition, ce capteur (26) étant en liaison avec un dispositif (27) pour la transmission sans fil à l'unité de suivi (11), des valeurs de capteur relevées.

18. Système de suivi selon l'une des revendications 1 à 17, **caractérisé en ce qu'**il est prévu un câble de liaison (28) pour relier l'unité de suivi (11) à un dispositif de communication, notamment à un ordinateur (29) ou à un téléphone.

19. Système de suivi selon l'une des revendications 1 à 18, **caractérisé en ce que** la deuxième chambre (5) de l'anneau gastrique (1) est formée par un port à installer en position sous-cutanée.

20. Système de suivi selon l'une des revendications 1 à 19, **caractérisé en ce que** les chambres (2, 5) de l'anneau gastrique sont reliées par l'intermédiaire d'une conduite (6), au moins un dispositif (10) pour commander le débit du fluide étant prévu dans la conduite (6).

21. Système de suivi selon l'une des revendications 1 à 20, **caractérisé en ce que** le dispositif de traitement de données (16) est conçu pour la détermination de la durée et de la vitesse d'une phase d'absorption de repas.

22. Système de suivi selon l'une des revendications 1 à 21, **caractérisé en ce que** le dispositif de traitement de données (16) est conçu pour évaluer l'amplitude et la durée des valeurs de pression ayant été déterminées pendant une phase d'absorption de repas.
